# EUROPEAN PATENT SPECIFICATION

(11) **EP 1 261 368 B1**
(45) Date of publication and mention of the grant of the patent: **28.04.2010**
(21) Application number: 01922251.2
(22) Date of filing: 23.02.2001
(51) Int. Cl.: A61K 38/46, A61K 38/47, A61K 38/48, A61P 1/00, A61P 3/00

(54) **CROSSLINKED, NON-FUNGAL LIPASE-CONTAINING COMPOSITION AND METHODS OF USE THEREOF**
VERNETZTE, NICHT VON PILZEN KOMMENDE LIPASE ENTHALTENDE ZUSAMMENSETZUNG UND METHODEN ZU DEREN VERWENDUNG
COMPOSITION CONTENANT UNE LIPASE NON-FONGIQUE RETICULEE ET SES PROCEDES D'UTILISATION

(30) Priority: 24.02.2000 US 184517 P; 22.02.2001 US 791947
(43) Date of publication of application: 04.12.2002
(73) Proprietor: Altus Pharmaceuticals Inc., Cambridge, MA 02139-4807 (US)
(72) Inventor: MARGOLIN, Alex, Newton, MA 02461 (US); SHENOY, Bhami, Woburn, MA 01801 (US)
(74) Representative: Vossius & Partner
(86) International application number: PCT/US2001/006074
(87) International publication number: WO 2001/062280

(56) References cited:
- WO-A-96/38170
- WO-A-98/46732
- WO-A-99/55310
- US-A- 4 079 125
- US-A- 5 614 189
- DATABASE WPI Section Ch, Week 199632 Derwent Publications Ltd., London, GB; Class A96, AN 1996-318858 XP002178605 & JP 08 143469 A (AMANO PHARM KK), 4 June 1996 (1996-06-04)
- MARGOLIN, A. L. ET AL: "Cross - linked enzyme crystals for lumenal therapies" PROC. INT. SYMP. CONTROLLED RELEASE BIOACT. MATER. (2000), 27TH, 1012-1013 , XP001024503

## Description

### FIELD OF THE INVENTION

This invention relates generally to compositions containing enzymes and more particularly to compositions containing lipase for the treatment of disorders characterized by low lipase secretion.

### BACKGROUND OF THE INVENTION

Metabolic or gastrointestinal diseases often result from the absence of an effective enzyme whose function is necessary at a particular point in a biochemical pathway. For example, improper lipase levels can be traced to a variety of digestive disorders, including fat malabsorption. Fat malabsorption often develops in patients suffering from cystic fibrosis, chronic pancreatitis, and other diseases of the pancreas when pancreatic lipase secretion falls below 5-10% of normal levels. Commonly observed consequences of fat malabsorption include abdominal discomfort, steatorrhea (fatty stools), essential fatty acid (EFA) deficiency, fat-soluble vitamin (*e.g*., A, D, E, and K) deficiency, and a generalized failure to thrive.

One recognized method for treating diseases or conditions associated with lipase insufficiency is oral replacement therapy. This treatment regimen includes orally administering lipase enzymes to an afflicted individual to increase digestion and a subsequent absorption of nutrients. Commercially available preparations of lipase can fail to completely treat symptoms associated with lipase insufficiency. For example, commercially available porcine lipase can fail to eliminate pancreatic steatorrhea caused by chronic pancreatitis or cystic fibrosis. Factors responsible for difficulties in the treatment of steatorrhea can include destruction of substituted lipase by gastric juice, destruction of substituted lipase by intraluminal proteases, and asynchronous gastric emptying of enzyme supplement and meal nutrients.

Lipases commonly used in replacement therapy are most active at an alkaline pH, and show significant loss of activity when the pH is less than 5. Pancreatic lipase, for example, has been reported to be irreversibly denatured at pH 4 or below. Because of this instability, lipase-based replacement therapies can include repeated administrations of lipases and/or administration of high doses of the enzymes to afflicted individuals. High doses of the enzymes can be associated with undesirable side effects.

### SUMMARY OF THE INVENTION

The invention is based in part on the discovery of compositions which include lipase in a crosslinked crystalline form that is highly resistant to proteolytic and acid degradation. Because the crosslinked crystalline lipase exhibits high stability against proteases and acid, the composition can be administered in low doses to patients suffering from gastrointestinal disorders. In one aspect the invention provides a composition that includes a crosslinked lipase crystal, a protease, and an amylase in amorphous form. The lipase crystal in the composition is crosslinked with a multifunctional crosslinking agent and is preferably stable at pH 1-9. The enzyme is active at a pH from about 2.0 to 9.0. More preferably, the enzyme is active at pH 4-7.

A preferred composition includes a cross-linked enzyme *Burkholderia cepacia* ( *"BC "*) crystal, a fungal or plant protease, and a fungal or bacterial amylase. Preferably, the protease is bromelain.

Preferably, the lipase crystal is active following exposure of the lipase crystal for extended periods of time to proteases, acidic conditions, elevated temperatures, or a combination thereof.

Also provided by the invention is a method for treating or preventing fat malabsorption in a mammal, *e.g*., a human, who suffers from, or is at risk for, a condition characterized by low lipase activity. The method includes administering to the subject a composition that includes a crosslinked crystal of a lipase, a protease and an amylase, in an amount sufficient to prevent or inhibit low lipase activity, or to reduce or prevent symptoms associated with low lipase activity.

The highly stable lipases described herein are stable upon administration to a subject. Thus, they can be administered in the absence of enteric-coated microsphere preparations. The lipases described herein can also be administered in lower doses to a subject.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

### BRIEF DESCRIPTION OF THE DRAWINGS

FIG. 1 is a histogram showing the effect of various doses of cross-linked *Burkholderia cepacia* enzyme complex ("CLEC-BC") on mean coefficient of fat absorption ("CFA").
FIG. 2 is a histogram showing the effect of various doses of CLEC-BC on mean stool fat.
FIG. 3 is a histogram showing the effect of various doses of CLEC-BC on mean coefficient of fat absorption ("CPA").
FIG. 4. is a histogram showing the effect of various doses of a particle containing CLEC-BC, amylase, and a protease on mean coefficient of fat absorption ("CFA").
FIG. 5 is a histogram showing the effect of various doses of a particle containing CLEC-BC, an amylase, and a protease on mean stool fat
FIG. 6 is a histogram showing the effect of various doses of a composition including CLEC-BC, an amylase and a protease on mean coefficient of protein absorption ("CPA").
FIG. 7 is a graph showing the effect of various therapeutic lipases on mean CPA.

### DETAILED DESCRIPTION OF THE INVENTION

The invention provides compositions including crosslinked lipase crystals that are unexpectedly active following exposure to harsh conditions associated with the upper gastrointestinal tract. These conditions include the acidic environment (*i.e.,* the low pH) of the stomach and high levels of proteases present in the gastrointestinal tract In preferred embodiments, the compositions are provided in long-lasting compositions that pass through the highly acidic gastric environment of the stomach and allow for delivery of the enzymes in the composition to the intestines of a subject.

The protease components can be provided in crystalline or amorphous, non-crystalline forms. The latter enzymes degrade carbohydrates and proteins present in the intestinal regions.

Because of the enhanced stability of the crosslinked lipase crystals, the pharmaceutical compositions of the invention have a higher specific activities in the gastrointestinal tract. As a result, they can be administered in lower amounts per dose, and can be administered fewer times over the course of a treatment regimen, at lower doses, and in fewer administrations.

### Compositions containing stabilized crosslinked lipase crystals

The invention provides a composition that includes a non-fungal lipase crystal, a protease and an amylase in amorphous form. The lipase crystal is present in the composition as a crosslinked crystal.

In general, any lipase can be used in the composition, as long as it can be provided in a crosslinked crystalline form that resists proteolytic degradation and is stable in low pH. In various embodiments, the lipase is provided as a crosslinked crystal that is stable at a pH less than 7, 6, 5, 4.5, 4, 3.5, 3.0, 2.5, 2.0,1.5 or less. The lipase can be isolated from a prokaryotic or a eukaryotic cell. Preferably, the lipase is from a non-fungal organism. A preferred source of the lipase is a *Pseudomonas* bacterium. If desired, the lipase can be isolated from a cell which expresses a recombinant form of the lipase.

Lipase crystals are grown by methods known in the art, *e.g*. by the controlled precipitation of protein out of aqueous solution, or aqueous solution containing organic solvents, as described in, for example, U.S. Patent No. 5,618,710. For example, lipase crystals can be produced by combining the lipase protein to be crystallized with an appropriate aqueous solvent or aqueous solvent containing appropriate precipitating agents, such as salts or organic agents. The solvent is combined with the lipase at a temperature determined experimentally to be appropriate for the induction of crystallization and acceptable for the maintenance of protein stability and activity. The solvent can optionally include co-solutes, such as divalent cations, cofactors or chaotropes, as well as buffer species to control pH. The need for and concentrations of co-solutes are determined experimentally to facilitate crystallization. In an industrial scale process, the controlled precipitation leading to crystallization can best be carried out by the simple combination of protein, precipitant, co-solutes, and optionally buffers in a batch process. Alternative laboratory crystallization methods, such as dialysis or vapor diffusion can also be adapted. For example, McPherson (Methods Enzymol. 114:112 (1985)), and Gilliland (J. Crystal Growth 90:51-59 (1988)) include a comprehensive list of suitable conditions in reviews of the crystallization literature. Occasionally, incompatibility between the cross-linking reagent and the crystallization medium might require exchanging the crystals into a more suitable solvent system.

Once crystals are grown in a suitable medium, they can be cross-linked. Cross-linking results in stabilization of the crystal lattice by introducing covalent links between the constituent enzyme molecules in the crystal. This makes possible the transfer of enzyme into an alternate reaction environment that might otherwise be incompatible with the existence of the crystal lattice, or even with the existence of intact undenatured protein. The cross-linking interactions prevent the constituent enzyme molecules in the crystal from going back into solution, effectively insolubilizing or immobilizing the enzyme molecules into microcrystalline structures.

The macroscopic, immobilized, insolubilized crystals can be readily separated from e.g., feedstock containing product or unreacted substrate by simple procedures known in the art, *e.g*, filtration and/or decantation.

Cross-linking can be achieved by a wide variety of reagents, *e.g*., glutaraldehyde. Cross-linking with glutaraldehyde forms strong covalent bonds between primarily lysine amino acid residues within and between the enzyme molecules in the crystal lattice that constitute the crystal. The crosslinking agent is a multifunctional crosslinking reagent. Crosslinking agents are described in, for example, the 1999 edition of the Pierce Chemical Company Catalog.

Examples of suitable crosslinking agents include glutareldehyde, succinaldehyde, octanedialdehyde and glyoxal. Additional multifunctional crosslinking agents include halotriazines, e.g., cyanuric chloride; halo-pyrimidines, e.g., 2,4,6-trichloro/bromo-pyrimidine; anhydrides or halides of aliphatic or aromatic mono- or di-carboxylic acids, e.g., maleic anhydride, (meth)acryloyl chloride, chloroacetyl chloride; N-methylol compounds, e.g., N-methylol-chloro acetamide; di-isocyanates or di-isothiocyanates, e.g., phenylene-1,4-diisocyanate and aziridines. Other crosslinking agents include epoxides, such as, for example, di-epoxides, tri-epoxides and tetra-epoxides. Such multifunctional crosslinking agents may also be used, at the same time (in parallel) or in sequence, with reversible crosslinking agents, such as dimethyl 3,3'-dithiobispropionimidate.HCl - (DTBP, Pierce), and dithiobis (succinimidylpropionate) (DSP, Pierce).

Formulations and compositions including crystals according to this invention may be crosslinked for additional stability. This allows for the use of such crystals, crystal formulations and compositions in areas of pH extremes, such as the gastrointestinal tract of humans and animals. For example, lipase crystals, may be crosslinked using one of a variety of crosslinkers, including, but not limited to, Dimethyl 3, 3'-dithiobis-propionimidate.HCl (DTBP), Dithiobis (succinimidylpropionate) (DSP), Bis maleimido- hexane (BMH), Bis[Sulfosuccinimidyl]suberate (BS), 1,5-Difluoro-2,4-dinitrobenzene (DFDNB), Dimethylsuberimidate.2HCl (DMS), Disuccinimidyl glutarate (DSG), Disulfosuccinimidyl tartarate (Sulfo-DST), 1-Ethyl-3-[3-Dimethylaminopropyl] carbodiimide hydrochloride (EDC), Ethylene glycolbis[sulfosuccinimidylsuccinate] (Sulfo-EGS), N-[g-maleimidobutyryloxy]succinimide ester (GMBS), N-hydroxysulfo-succinimidyl-4-azidobenzoate (Sulfo-HSAB), Sulfosuccinimidyl-6-[a-methyl-a-(2-pyridyldithio)toluamido] hexanoate (Sulfo-LC-SMPT), Bis-[b-(4-azidosalicylamido) ethyl]disulfide (BASED) and glutaraldehyde (GA).

In some embodiments, the lipase crystal is provided as a crystal in a powder form. The powder form can be produced, for example, by lyophilization or spray-drying. Lyophilization, or freeze-drying, allows water to be separated from the composition, producing a crystal which can be stored at non-refrigerated (room) temperatures for extended periods of time, and which can be easily reconstituted in aqueous, organic, or mixed aqueous-organic solvents of choice without the formation of amorphous suspensions and with minimal risk of denaturation. Carpenter, et al., Pharm. Res., 14:969 (1997). Lyophilization may be performed as in U.S. Patent No. 5,618,710, or by any other method known in the art. For example, the protein crystal is first frozen and then placed in a high vacuum where the crystalline water sublimes, leaving a protein crystal behind which contains only the tightly bound water molecules.

Because the cross-linked lipases described herein are stable against proteases, the preparations can be formulated in water and provided as aqueous slurry formulations, which is a preferred mode of administering lipases, especially to a pediatric subject.

The catalytic activity of crystallized lipase can be measured using any method known in the art. For example, lipase activity can be determined spectrophotometrically as described in Example 6 of U.S. Patent No. 5,618,710. Lipase activity can be determined by monitoring hydrolysis of the substrate p-nitrophenyl acetate. Substrate cleavage is monitored by increasing absorbance at 400 nm, with an initial substrate concentration of 0.005% and starting enzyme concentration of 1.5 X 10⁻⁸ M. Lipase enzyme is added to a 5 ml reaction volume containing substrate in 0.2 M Tris pH 7.0 at room temperature. Crystalline lipase is removed from the reaction mixture by centrifugation prior to measuring absorbance.

Alternatively, lipase activity can be measured *in vitro* by hydrolysis of olive oil as described in Examples 2-4 of U.S. Patent No. 5,614,189.

Lipase activity can also be measured *in vivo.* For example, a small volume (about 3 ml) of olive oil or corn oil can be labeled with ⁹⁹TC-(V) thiocyanate, and crystalline lipase can be labeled with ¹¹¹¹In. The labeled fat is mixed with an animal food on to which is sprinkled the labeled crystalline lipase. Scintigraphic images of the proximal and distal stomach and small intestine are obtained until <5% of the activity remains in the stomach. Emptying curves for each of the isotopes (*e.g*., percent retention in the stomach over time) and amounts of isotopes entering the proximal, middle, and distal small bowel from the respective regions of interest are determined.

Preferably, the composition includes a crosslinked crystalline lipase that has a high specific activity. A high specific activity lipase activity is typically one that shows a specific activity to triolein (olive oil) at greater than 500, 1000, 4000, 5000, 6000, 7000, 8000, or 9000 or more units/mg protein.

A preferred lipase is also stable for an extended period of time in a harsh environment found in gastrointestinal regions, *e.g*., gastric, duodenal and intestinal regions. For example, the lipase is preferably stable for at least one hour in acidic pH, *e.g.,* an environment in which the pH is less than 7, 6, 5, 4.5, 4, 3.5, 3.0, 2.5, 2.0, 1.5 or less.

Alternatively, or in addition, the crosslinked crystalline lipase crystal in the composition is heat resistant. For example, in various embodiments, the crosslinked crystalline lipase in various embodiments is stable for at least one hour at 30°C, 35°C, 37°C, 40°C, 42°C or even 45°C.

Preferably, the composition is stable in the harsh environment, *e.g*., the acidic environments or high temperature environments, or both, for at least 1, 2, 3, 4, 5, 6, or 12 or more hours.

By "stable" is meant that the lipase crystal is more active than the soluble form of the lipase for the given condition and time. Thus, a stable lipase crystal retains a higher percentage of its initial activity than the corresponding soluble form of the lipase. In some embodiments, the lipase crystal is more active than the non-cross-linked crystalline form of the lipase. In some embodiments, the lipase crystal retains at least 50% of its activity after exposure to the given conditions and time. In some embodiments, the lipase retains 60%, 65%, 75%, 85%, 90%, or more of its activity.

The lipase crystal in the composition is active following exposure for at least one hour to an environment having a pH from 1.0 to 4.0, Alternately, the lipase crystal in the composition is active following exposure for at least two hours to an environment having a pH from 1.0 to 4.0. Alternately, the lipase crystal in the composition is active following exposure for at least five hours to an environment having a pH from 1.0 to 4.0.

The composition is provided with a protease and an amylase. Any protease known in the art can be use in the composition. Preferred proteases are trypsin, bromelain, papain, fungal proteases, or a combination of these proteases.

The amylase can be from any suitable prokaryotic or eukaryotic host. Preferred amylases include those from *Bacillus* or *Aspergillus* species.

Additionally, either the protease, amylase, or both, may be provided in the crystalline form or in a lyophilized form. While the protease, amylase, or both, can be provided in the lyophilized form, in preferred embodiments these are present in non-crystalline, *i.e*., amorphous, forms.

If desired, additional components can be present in the composition. These components can include, *e.g*., an esterase.

### Pharmaceutical compositions containing acid-stable crosslinked lipase crystals, a protease, and an amylase

Included in the invention is a pharmaceutical composition which includes an acid stable, proteolytic-resistant lipase, a protease and an amylase. The lipase is provided in a crosslinked crystalline form.

The term "pharmaceutically acceptable" means approved by a regulatory agency of the Federal or a state government or listed in the U.S. Pharmacopoeia or other generally recognized pharmacopoeia for use in animals and, more particularly, in humans. The term "carrier" refers to a diluent, adjuvant, excipient, or vehicle with which the therapeutic is administered.

Typical excipients, include sugars and biocompatible polymers. Examples of excipients are described in the *Handbook of Pharmaceutical Excipients,* published jointly by the American Pharmaceutical Association and the Pharmaceutical Society of Great Britain. Representative excipients include sucrose, trehalose, lacitol, gelatin, hydroxypropyl-β-cyclodextrin, methoxypolyethylene glycol, and polyethylene glycol.

If the composition is to be provided in capsule or tablet form, a diluent may be included. Typical diluents include, *e.g*., calcium carbonate, dibasic calcium phosphate, tribasic calcium phosphate, calcium sulfate, microcrystalline cellulose, powdered cellulose, dextrates, dextrin, dextrose excipient, fructose, kaolin, lactose, mannitol, sorbitol, starch, pregelatinized starch, sucrose, compressible sugar, confectionery sugar. Preferably, the pharmaceutical composition is formulated for oral delivery.

In some embodiments, the lipase, protease, and amylase composition is present in the pharmaceutical composition in association with a polymeric carrier. In one embodiment, a slow release composition containing a cross linked crystal lipase is formed. The formulation of crosslinked lipase crystals, lyophilized amylase, lyophilized protease and a polymeric carrier allows for an acid-resistant controlled release capsule that results in delivery of the enzymes in effective amounts and at low doses to the intestine, *e.g*., the distal bowel, following oral ingestion. Furthermore, lipase crystals encapsulated within polymeric carriers to form microspheres can be dried by lyophilization.

A polymeric carrier can include, *e.g*., polymers used for encapsulation of protein crystals for delivery of proteins, including controlled release biological delivery. Such polymers include biocompatible and biodegradable polymers. Preferably, the polymeric carrier is a biodegradable polymer. Biodegradable polymers are polymers that degrade by hydrolysis or solubilization. Degradation can be heterogeneous, *i.e*., occurring primarily at the particle surface, or homogenous, *i.e*., degrading evenly throughout the polymer matrix, or a combination of such processes. The polymeric carrier may be a single polymer type or it may be composed of a mixture of polymer types.

To protect the lipase, protease, and amylase from the harsh environment of the gastrointestinal tract, the composition is preferably encapsulated within a matrix of the polymeric carrier.

Microspheres are produced when protein crystals are encapsulated in at least one polymeric carrier to form microspheres by virtue of encapsulation within the matrix of the polymeric carrier to preserve their native and biologically active tertiary structure. The crystals can be encapsulated using various biocompatible and/or biodegradable polymers having unique properties which are suitable for delivery to different biological environments or for effecting specific functions. The rate of dissolution and, therefore, delivery of active protein is determined by the particular encapsulation technique, polymer composition, polymer crosslinking, polymer thickness, polymer solubility, protein crystal geometry and degree and, if any, of protein crystal crosslinking. The crystal(s) may be encapsulated using a variety of polymeric carriers having unique properties suitable for delivery to different and specific environments or for effecting specific functions. The rate of dissolution of the compositions and, therefore, delivery of the active protein can be modulated by varying crystal size, polymer composition, polymer crosslinking, crystal crosslinking, polymer thickness, polymer hydrophobicity, polymer crystallinity or polymer solubility.

In some embodiments, the pharmaceutical composition is provided as a controlled release composition. For example, the composition can be one in which at least 25%, 50%, 75%, 80%, 85%, 90%, or even 95% or more of the composition remains encapsulated within the matrix following exposure of the polymeric carrier to an acidic environment for an extended period of time, *e.g*, an acidic environment having a pH less than 7, 6, 5, 4.5, 4, 3.5, 3.0,2.5,2.0, 1.5, or less for at least one hour. In some embodiments, the composition is retained in the acidic conditions for 2, 3, 4, 6, 10, 12, or 24 or more hours. For oral delivery the composition can also be one in which at least 50% of the composition remains encapsulated within the matrix following exposure of the polymeric carrier to an environment having a pH from 1.0 to 3.0 for at least one hour.

In various embodiments, the pharmaceutical composition is to be administered to a subject prior to, simultaneous with, or following ingestion of food by the subject. The subject to which the composition is to be administered preprandially, prandially, or postprandially can be, *e.g.,* a human, dog, cat, mouse, rat, horse, cow, or other mammal.

### Therapeutic uses for compositions containing stabilized crosslinked lipase crystals

Also provided by the invention are methods for treating or preventing gastrointestinal disorders in a mammal. According to this method, a therapeutically effective to amount of a crosslinked crystalline lipase, protease, amylase composition is administered to a subject in need of treatment. The subject to can be *e.g.,* a human, dog, cat, mouse, rat, horse, cow, or other mammal. Preferably, the composition is administered orally, *e.g.,* at mealtime. For example, the composition can be administered just before, just after, or while eating.

The compositions of the invention can be used to treat or prevent, for example, pancreatitis, pancreatic insufficiency, fat malabsorption, low lipase secretion, and gastrointestinal complications associated with cystic fibrosis. The methods of this invention can be also be used to treat any condition characterized by inadequate amounts of or ineffective lipase. Such conditions include steatorrhea, essential fatty acid deficiency, failure to thrive, and fat-soluble vitamin deficiency.

The effectiveness of the method of treatment can be assessed by measuring and comparing the coefficient of fat absorption (CFA) in healthy individuals with that of the subject being treated according to the methods of this invention. For example, a healthy mammal has a CFA greater than 90%. Subjects suffering from a gastrointestinal disorder characterized by pancreatic deficiency, pancreatitis, fat malabsorption or low lipase secretion, will typically have a CFA of less than 60%. Preferably, the methods of this invention are employed to increase the CFA of a subject in need of treatment to at least 60%. More preferably, the CFA is increased to greater than 80%. Most preferably, the CFA is increased to greater than 85%.

An alternative means for measuring the efficacy of treatment of a subject according to the methods of this invention is by performing a 72 hour stool test. For example, effective treatment according to the invention decreases stool fat content in an adult human subject to less than 7 grams a day.

The invention will be further illustrated in the following non-limiting examples.

### Example 1. US Pharmacopeia Assay for lipase activity

The activity of *Burkholderia cepacia* lipase was determined by titrating the released fatty acids from olive oil against sodium hydroxide as described by U.S. Pharmacopeia (Assay for lipase activity in Pancreatin, USP 24, 2000, 1254-1255). The lipase activity in USP units was calculated by comparison to the activity of the standard, using the lipase activity stated on the label of USP Pancreatin Lipase RS. One USP unit of lipase activity is the amount of enzyme that liberates 1.0µ Eq of acid per minute at pH 9.0 and 37°C under the conditions of the Assay for lipase activity.

### Example 2. Olive oil-based assay for lipase activity

Lipase activity was measured using an olive oil assay. Lipase supernatant sample were assessed for activity against olive oil in pH 7.7 buffer. The assay was carried out titrimetrically using slight modifications to the procedure described in Pharmaceutical Enzymes - Properties and Assay Methods, Ruyssen and Lauwers, (Eds.), Scientific Publishing Company, Ghent, Belgium (1978).

Solutions used in the assay included the following:
1. Olive oil emulsion: 16.5 gm of gum arabic (Sigma) was dissolved in 180 ml of water and 20 ml of olive oil (Sigma) and emulsified using a Quick Prep mixer for 3 minutes.
2. Titrant : 0.05 M NaOH;
3. Solution A: 3.0 M NaCl;
4. Solution B: 75 mM CaCl₂.2H₂O;
5. Mix: 40 ml of Solution A was combined with 20 ml of Solution B and 100 ml of H₂O;
6. 0.5% Albumin.

### Lipase Substrate Solution

The substrate was prepared by adding 50 ml of olive oil emulsion (solution 1) to 40 ml of Mix (solution 5) and 10 ml of 0.5% albumin (solution 6).

### Assay Procedure

The lipase substrate solution (solution 7) was warmed to 37°C in a water bath. First, 20 ml of substrate was added to a reaction vessel and the pH was adjusted to 7.7 using 0.05 M NaOH (solution 2) and equilibrated to 37 °C with stirring. The reaction was initiated by adding enzyme. The reaction progress was monitored by titrating the mixture of enzyme and substrate with 0.05 M NaOH to maintain the pH at 7.7.

The specific activity (µmoles/min/mg protein) was equal to the initial rate X 1000 X concentration of the titrant/the amount of enzyme. The zero point was determined by running the reaction without enzyme, i.e., using buffer in the place of enzyme in the reaction mixture. The initial rate was equal to base consumption in ml/ time in min. The blank was a sample without enzyme, i.e., buffer was used instead of enzyme in the reaction mixture.

### Example 3. Assay for protease activity

The activity of proteases was determined by using casein as a substrate in a procedure as described by U.S. Pharmacopeia (Assay for protease activity in Pancreatin, USP 24, 2000, 1254-1255). The protease activity in USP units was calculated by comparison to the activity of the standard, using the protease activity stated on the label of USP Pancreatin Amylase and Protease RS. One USP unit of protease activity is the amount of enzyme that hydrolyzes casein at an initial rate such that an amount of peptide (that is not precipitated by trichloroacetic acid) is liberated per minute that gives the same absorbance at 280 nm as 15 nmol of tyrosine under the conditions of the assay for protease activity.

### Example 4. Assay for amylase activity

The activity of amylases was determined using starch as substrate as described by U.S. Pharmacopeia (Assay for amylase activity in Pancreatin, USP 24, 2000, 1254-1255). The amylase activity in USP units was calculated by comparison to the activity of the standard, using the amylase activity stated on the label of USP Pancreatin Amylase and Protease RS. One USP unit of amylase activity is the amount of enzyme that decomposes starch at an initial rate such that 0.16 mEq of glycosidic linkage is hydrolyzed per minute under the conditions of the Assay for amylase activity.

### Example 5. Crystallization of Burkholderia cepacia lipase

The lipase from *Burkholderia cepacia* (lipase PS 3000 - Amano) ("LPS", 150 gm) was dissolved in 1000 mL distilled deionized water and dialyzed against water overnight with three changes. To the protein, tert-butanol was added to a final concentration of 25% and 1M sodium acetate buffer was added to a final concentration of 10 mM, followed by centrifugation to remove the precipitate that had formed after 1 hour. The crystals of lipase started forming in 15 min. Crystallization was then allowed to proceed for 16 hrs before harvesting. More than 95% yield (based on activity) was obtained by this procedure.

The crystals were rod shaped and fairly uniform in size (approximately 10-15 µm in length) and shape when observed under light microscope, and as measured by a Coulter LS Particle size analyzer.

### Example 6. Crosslinking of Burkholderia cepacia lipase crystals

Crosslinking of lipase crystals was carried out using 2 mM Bis (Sulfosuccinimidyl) suberate BS ("BS") in mother liquor (25% tert-butanol at pH 8.5 in 50mM phosphate buffer). Crosslinking was carried out at 4 °C overnight (16hrs) with tumbling. After 16 hours, the slurry was centrifuged at 3000 rpm and the supernatant was discarded. The crosslinking was terminated by washing off excess reagent with mother liquor in the presence of 10 mM Tris.HCl to inactivate the any unreactive cross-linker. Finally, the cross-linked *Burkholderia cepacia* enzyme complex (CLEC-BC) was washed thoroughly with 10 mM sodium acetate buffer, pH 4.5 and stored at 4°C.

### Example 7. Measuring crystallinity

The crystal integrity of the formulations was monitored by inspection under a light microscope and by Coulter counter analysis for particle size measurement. The rod shape of the crystals and their size remained unchanged after crosslinking.

### Example 8. Secondary structure characterization by Fourier transform infrared (FTIR) spectroscopy

The Fourier transform infrared (FTIR) spectra of soluble, crosslinked, and noncrosslinked lipase crystals were collected on a Nicolet model 550 Magna series spectrometer as described by Dong et al. in Biochemistry 31:9364-70 (1992) and in J. Pharm. Sci.: 84:415-24 (1995). The noncrosslinked lipase crystal slurry and CLEC-BC slurry samples (about 5 to 10 mg/ml each) were placed on a Zinc selenide crystal of ARK ESP. The spectra were collected and processed using Grams 32 from Galactic Software for the determination of relative areas of the individual components of secondary structure using second derivative and curve-fitting program under the amide I region (1600 -1700 cm⁻¹). Both noncrosslinked soluble lipase and CLEC-BC gave identical spectra without any major changes in secondary structure.

### Example 9. Activity of cross-linked enzyme Burkholderia cepacia ("BC") crystals

The activity of cross-linked enzyme *Burkholderia cepacia* ("BC") crystals was examined.

CLEC-BC crosslinked with Bis (Sulfosuccinimidyl) suberate (BS) is active. The CLEC-BC crosslinked with BS was approximately ∼50% active when compared to noncrosslinked soluble lipase (Table 1). The activities of the soluble lipase and the crosslinked lipase were compared using both the USP method (Example 1) and olive oil-release (Example 2) method.

**Table 1. Activity of lipase CLEC preparation**

| Sample | Specific Activity (Units/mg) |
|---|---|
| 1. Noncrosslinked (Soluble) lipase (USP assay) | 7184 |
| 2. CLEC-BC (olive oil assay) | 3010 |
| 3. CLEC-BC (USP assay) | 1727 |
| | |

### Example 10. Activity of CLEC-BC at various pH levels

The activity of the CLEC-BC was determined at various pH levels using end point titration. The activities were determined at pH 2.0, pH 4.5, pH 5.5, pH 6.5, pH 7.7 and pH 9.0. The samples were titrated using pH STAT for 15 min at the above-mentioned pH levels and then the pH of each sample was immediately raised to pH 7.7, except for the pH 9.0 sample, which was measured as it was. In the cases where the pH was raised to 7.7, the controls were run immediately without incubation for 15 minutes.

CLEC-BC crosslinked with Bis (Sulfosuccinimidyl) suberate-BS was active at various pH levels tested. The CLEC-BC showed activity at various pH ranges. With the exception of pH 2.0, at which only 25% activity was observed, CLEC-BC showed high activity at all pH levels tested.

### Example 11. Stability of CLEC-BC over time

The stability of CLEC-BC over time was examined. Stability of the CLEC-BC was determined at different pH levels at 37 °C for 5 hours. The CLECs were suspended in pH 2.0 (glycine•HCl buffer), pH 3.0 (glycine•HCl buffer), pH 4.0 (acetate buffer), pH 5.0 (acetate buffer), pH 6.0 (phosphate buffer), pH 7.0 (phosphate buffer), pH 8.0 (phosphate buffer), pH 9.0 (carbonate bicarbonate buffer), and pH 10.0 (carbonate bicarbonate buffer) separately for 5 hrs at 37 °C. Stability of the CLECs was determined by estimating the activities of the CLECs at time zero and at the end of 5 hours. Stability of soluble BC enzyme was also examined at pH 2.0 over a time period of five hours. Activity was measured as a percentage of starting activity.

CLEC-BC was found to be stable at all pH values tested during the time range examined.

### Example 12. Measurement of crystal dissolution in buffer

The solubility of a BC-CLEC formulation was determined under acidic conditions using 10 mM glycine•HCl buffer, pH 2.0. The CLECs were washed with 10 mM glycine•HCl buffer, pH 2.0, and suspended in the same buffer with tumbling at 37 °C for 5 hr. The crystal dissolution was examined by passing an aliquot through a 0.22 um filter. Protein (Bradford's method) and lipolytic activity (lipase assay using olive oil) were determined in the filtrate (Soluble CLEC) which gave the amount of crystals solubilized. For determining the activity of the crystals (CLEC), the soluble enzyme activity was subtracted from the total activity in the sample (Activity before filtration).

No significant leaching from CLEC-BC was observed at pH 2.0. The solubility of the CLEC formulation was determined under acidic conditions using 10 mM glycine.HCl buffer, pH 2.0. The CLECs were washed with 10 mM glycine.HCl buffer, pH 2.0, and suspended in the same buffer with tumbling at 37°C for 5 hr. Only 0.44% leaching was observed over a period of 5 hours.

### Example 13. In vitro assay of bioavailability of CLEC-BC

Stability against proteolytic degradation was assessed by incubating the CLEC with various proteases, such as pepsin (which is present in the stomach), and trypsin or chymotrypsin (which are present in the duodenum). In addition, protease bromelain was tested because it had been selected to be included in a combination therapy to substitute for protease in the pancreatic extract. Each CLEC was incubated at 37°C under gentle agitation in a solution of either 10 mM glycine•HCl buffer, pH 2.0 for pepsin or 10 mM phosphate buffer for trypsin/chymotrypsin, pH 7.0 or 10 mM acetate buffer, pH 5.5 for bromelain with a CLEC to protease ratio of 10:1 1 (W/W). Aliquots were taken at every hour and measured for the residual lipolytic activity using olive oil as substrate.

CLEC-BC showed high stability against pepsin treatment for 5 hours, without any loss of activity or crystal lattice. Under similar conditions, the soluble lipase lost about 58% activity. CLEC-BC showed no loss in activity after 5 hours incubation with trypsin, while soluble enzyme lost about 36% activity in 4 hr under similar conditions. With chymotrypsin, CLEC-BC showed only 28% loss in activity for 5 hours, while soluble lipase lost 82% of activity in 5 hours at pH 7.0. In addition, both CLEC-BC and soluble lipase were stable to proteolytic degradation by bromelain.

### Example 14. In vivo assay of bioavailability of CLEC-BC

Efficacy and bioavailability studies were performed to demonstrate that administration of particles (5-20 µm diameter) of CLEC-BC will correct steatorrhea in canines with pancreatic insufficiency. Reduction of steatorrhea with CLEC-lipase is related to survival of lipolytic activity. Slowing of gastric emptying, which occurs with high fat meals, enhances the mixing of the lipase with fat that leads to efficient fat digestion and absorption.

For the studies, female mongrel dogs weighing between 18-21 kg were used. Dogs were first anesthetized with an intravenous injection of thiopental sodium and then underwent endotracheal intubation. Anesthesia was maintained by halothane gas. After celiotomy, both the minor and major pancreatic ducts were individually ligated, and all other tissue connections between the duodenum and the head of the pancreas were transected.

During the balance studies, the dogs were fed two meals a day. With the first meal of the study, a carmine red marker was given. After appearance of carmine red in stool, a 72-hour stool collection was started. Fecal consistency (Grade 1: well-formed, Grade 2: mushy or loose, Grade 3: watery) and frequency were recorded and a fecal score was calculated by multiplying fecal consistency and frequency. The 72-hour stool was analyzed for total weight, carbohydrate, fat, and protein. Between studies, the dogs were maintained as described below for at least 3-7 days before beginning another fecal balance study.

Each dog ingested a high fat meal containing 850 Kcal comprised of 21, 43 and 36% of calories, respectively, as carbohydrate, fat and protein. The basic meal was Hill's canned dog food (Hill's Pet Products, Topeka, KS). It contained chicken, meat by-product, rice, ground corn, liver, animal fat, whole egg, turkey, soybean meal and cracked pearled barley. The meal was supplemented with 46-g promod powder and minerals. A high fat meal (high fat, high protein, and low carbohydrate) was used. In addition, this meal was associated with the best coordination between solid meal emptying and lipase delivery to the duodenum. Suzuki et al., Gastroenterology 112:2048-55 (1997); Cornell, Experiments with mixtures New York: Wiley, (1981); Boivin et al., Gastroenterology 99:1763-1771(1990). To adjust the mineral content so that the mineral requirement per day was nearly equal among the meals, 1.7g Na3(C6H507) and 2.0g KCl was added to the meal. Overall content of mineral was as follows: Ca-2088 mg, Na-1170mg, K-2108mg, C1-1869mg, P-1699mg, Mg-180mg.

Between studies, dogs were fed canned dog food (Hill's prescription diet, canine i/d, Hill's Pet Products, Topeka, KS). Each can contained 580 Kcal, comprised of 48% carbohydrate, 27% fat and 25% protein as percentage of calories, 15 g of fat as triglyceride, diglyceride, monoglyceride and fatty acid, 1 g of cholesterol and 1 g of cholesterol ester, and 0.5 g of phospholipid. Dogs were fed two cans in the morning and one can in the afternoon. Ten grams of porcine pancreatin powder (Viokase, AH Robins Company, Richmond, VA) were given with the morning meal and 7 g with the afternoon meal. This dose of pancreatic enzymes maintains the body weight of pancreatic insufficient dogs within 10% of preoperative values. Dogs were weighed weekly. Fasting blood glucose levels were measured weekly.

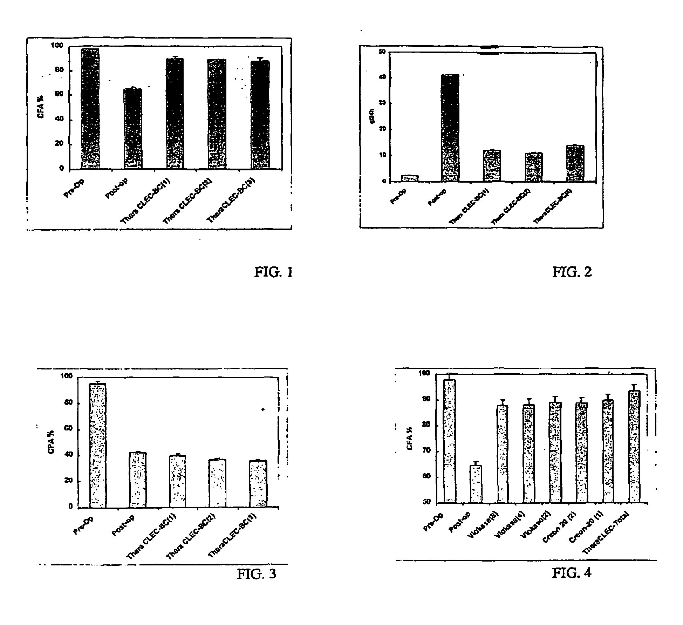

The results are presented in FIGS. 1, 2, and 3. CLEC-BC was administered at doses of 150,000 units ("Thera CLEC-BC" (1)" in FIGS. 1-3); 30,000 units ("Thera CLEC-BC" (2) in FIGS. 1-3), or (7,500 units "Thera CLEC-BC" (3) in FIGS. 1-3). FIG. 1 shows the effect of various doses of CLEC-BC on mean coefficient of fat absorption (CFA). Post-operative mean CFA levels in untreated dogs was reduced to about 60% of pre-operative levels. For all doses tested, addition of CLEC-BC restored percent CFA to about 90% of pre-operative levels.

FIG. 2 shows the effect of various doses of CLEC-BC on mean stool fat. In untreated post-operative dogs, mean stool fat increased from barely detectable levels to 40 grams/24 hours. Addition of CLEC-BC to post-operative dogs decreased mean stool fat to about 10 grams/24 hours for all doses tested.

FIG. 3 shows the effect of various doses of CLEC-BC on mean coefficient of protein absorption (CPA) in four dogs. Mean CPA decreased from about 95% absorption in pre-operative dogs to about 40% in post-operative dogs. Addition of CLEC-BC did not significantly affect mean CPA. CLEC-BC achieved reductions in CFA comparable to those observed using VIOKASE® and CREON®, two agents used to treat pancreatic exocrine insufficiency.

However, CLEC-BC differed from the agents in the amount of dose required to correct steatorrhea in dogs. Similar effects were achieved by using only 6-113 mg CLEC-BC vs. 1-4 g VIOKASE® and 0.5-1.0 g of CREON®. It can be seen from FIG. 3 CLEC-BC did not increase CPA over its postoperative, untreated level.

### Example 15. In vivo assay of bioavailability of a composition including CLEC-BC, bromelain, and amylase

The bioavailability of a composition including CLEC-BC, bromelain, and amylase in correcting pancreatic azotorrhea was examined. Efficacy was compared to efficacy of addition of the lipases VIOKASE® and CREON®.

The coefficient of protein absorption (CPA) following addition of the agents was measured by 72-hr fecal balance studies immediately before and after the operation resulting in pancreatic insufficiency, and 3 weeks after the operation with the doses of the following products: VIOKASE® 8, 4 and 2 tablets (240,000 120,000 and 60,000 USP protease units); CREON-20® 2 and 1 capsules (150,000 and 75,000 USP of protease); CLEC-Total consisting of CLEC-BC (150,000 USP; 113.5 mg), bromelain (150,000 USP; 214mg) and *Bacillus* amylase (150,000 USP; 71.9 mg). The enzyme activity of these preparations is shown in Table 2.

During these studies, dogs ingested a diet comprised of 43% fat, 36% protein and 21% carbohydrate as a percentage of calories (1700 kCal/d).

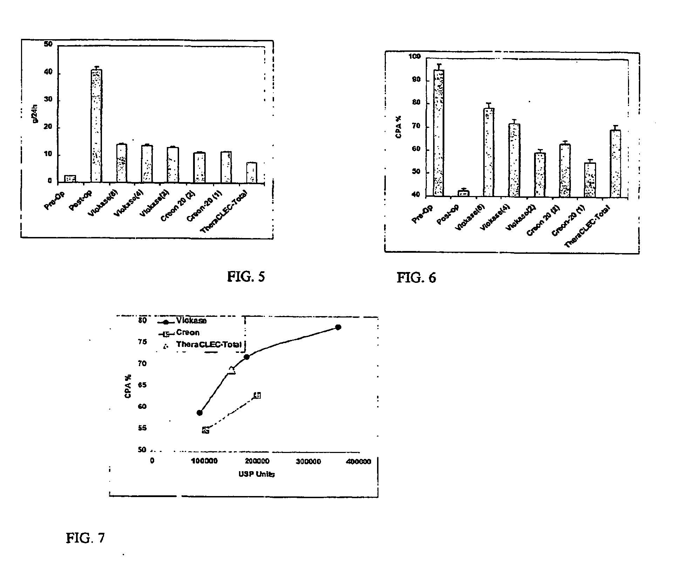

The coefficient of fat absorption was > 88 % with all treatments (FIG. 4). The coefficient of protein absorption (CPA) was directly correlated (r = 0.86) with the amount of protease in the preparations. The CPA increased from 59% with the lowest protease dose to 79% with the highest protease dose. The CPA with bromelain (69%; 150,000 USP Protease) was similar to the CPA of 2 capsules of CREON® (63%; 150,000 USP units) and to the CPA of 4 tablets of VIOKASE® (72%; 120,000 USP protease units) (FIGS. 6 and 7). It was noted that the actual proteolytic activities of the VIOKASE® and CREON® were at least 35% higher than their stated activities. Indeed, the actual protease activity of VIOKASE® was 46,248 USP units per tablet vs the 30,000 USP units per tablet claimed for VIOKASE® , and 100,519 USP units per capsule vs the 75,000 USP units per capsule claimed for CREON® .

**Table 2. Activities of lipase, protease and amylase in various therapeutic preparations.**

| | Number of Tablets/Capsules | Weight (mgs) | Lipase (USP units) | CFA % | Protease (USP units) | CPA % | Amylase (USP units) |
|---|---|---|---|---|---|---|---|
| Viokase | 8 | 3440 | 64,000 | 88 | 369,984 | 79 | 404,352 |
| | 4 | 1720 | 32,000 | 88 | 184,992 | 72 | 202,176 |
| | 2 | 860 | 16,000 | 89 | 92,496 | 59 | 101,088 |
| Creon® | 2 | 944 | 40,000 | 89 | 201,038 | 63 | 219,420 |
| | 1 | 472 | 20,000 | 90 | 100,519 | 55 | 109,710 |

These results demonstrate that a composition containing CLEC-BC, bromelain, and amylase is at least as effective on a per unit basis as porcine proteases in reducing protein malabsorption (FIG. 6 and 7). In addition, the results show that a small amount (114 mg) of CLEC-BC in CLEC-Total corrects steatorrhea in dogs at least as well as 8 tablets of VIOKASE® (∼ 4 gm) or 2 capsules of CRESON (∼1 gm) (FIG. 4).

Other embodiments are within the claims.

## Claims

1. A composition comprising:
(a) a non-fungal lipase crystal crosslinked with a multifunctional crosslinking.agent;
(b) a protease; and
(c) an amylase, in amorphous form
wherein the lipase crystal is active at a pH from 2.0 to 9.0.

2. The composition according to claim 1,
wherein the lipase crystal is active following exposure for at least one hour to an environment having a pH from 1.0 to 4.0.

3. The composition according to claim 1,
wherein the lipase crystal is active following exposure for at-least two hours to an environment having a pH from 1.0 to 4.0.

4. The composition according to claim 1,
wherein the lipase crystal is active following exposure for at least five hours to an environment having a pH from 1.0 to 4.0.

5. The composition according to claim 1
wherein the multifunctional crosslinking agent is Bis (Sulfosuccinimidyl) suberate.

6. The composition according to claim 1
wherein the lipase is derived from a bacterial lipase.

7. The composition according to claim 6, wherein the bacterial lipase is.a *Pseudomonas* lipase.

8. The composition according to claim 7, wherein the *Pseudomonas* lipase is *Burkholderia cepacia* lipase.

9. The composition according to claim 1, wherein the amylase is selected from the group consisting of a *Bacillus* amylase and an *Aspergillus* amylase.

10. The composition according to claim 1
wherein the protease is a fungal protease.

11. The composition according to claim 1
wherein the protease is a protease crystal.

12. The composition according to claim 11, wherein the protease crystal is a crosslinked protease crystal.

13. The composition according to claim 1
wherein the protease is in non-crystalline form.

14. The composition according to claim 1
wherein the composition is in a powder form.

15. The composition according to claim 1
wherein the composition is an aqueous slurry.

16. The composition according to claim 1
wherein the composition is in a capsule or tablet form.

17. The compositions according to claim 1
wherein the lipase crystal shows a specific activity to olive oil at greater than 500, 1000, 4000, 5000, 6000, 7000, 8000, 9000 or more units per mg lipase.

18. The composition according to claim 1 further comprising a pharmaceutically acceptable carrier.

19. The composition according to claim 1
wherein the composition is a pharmaceutical composition.

20. The composition according to any one of claims 1, 18 or 19, wherein the composition is present in a formulation suitable for oral delivery to a subject.

21. The composition according to claim 18, wherein the pharmaceutical carrier is selected from the group consisting of a diluent, excipient, and adjuvant.

22. The composition according to claim 18, wherein the carrier is a polymeric carrier.

23. The composition according to claim 22, wherein the polymeric carrier is a biodegradable polymer.

24. The composition according to claim 22, wherein the composition is encapsulated within a matrix of the polymeric carrier.

25. The composition according to claim 24, wherein at least 50% of the composition remains encapsulated within the matrix following exposure of the polymeric carrier to an environment having a pH from 1.0 to 3.0 for at least one hour.

26. Use of the composition according to claim 1 for the preparation of a pharmaceutical composition for treating or preventing a gastrointestinal disorder in a mammal.

27. The use according to claim 26, wherein the composition is to be administered orally.

28. The use according to claim 26, wherein the mammal is a human.

29. The use according to claim 26, wherein the gastrointestinal disorder is selected from the group consisting of pancreatitis and pancreatic insufficiency.

30. The use according to claim 26, wherein the subject suffers from or is at risk for cystic fibrosis.

31. Use of the composition according to claim 1 for the preparation of a pharmaceutical composition for treating or preventing fat malabsorption in a mammal suffering from or at risk for a condition **characterized by** low lipase secretion.

32. The use according to claim 31, wherein the composition is to be administered orally to the mammal.

33. The use according to claim 31, wherein the mammal is a human.

34. The use according to claim 31, wherein the composition is to be administered preprandially to the subject.

35. The use according to claim 31, wherein the composition is to be administered prandially to the subject.

36. The use according to claim 31, wherein the composition is to be administered postprandially to the subject.

37. The use according to claim 31, wherein the composition is to be administered to the mammal in an amount sufficient to increase the coefficient of fat absorption in the mammal to greater than 60%.

38. The use according to claim 31, wherein the composition is to be administered to the mammal in an amount sufficient to increase the coefficient of fat absorption in the mammal to greater than 80%.

39. The use according to claim 26, wherein the composition is to be administered to the mammal in an amount sufficient to increase the coefficient of protein absorption in the mammal to greater than 60%.

## Patentansprüche

1. Zusammensetzung umfassend:
(a) einen nicht von Pilzen stammenden Lipasekristall vernetzt mit einem multifunktionalen Vernetzungsmittel;
(b) eine Protease; und
(c) eine Amylase in amorpher Form,
wobei der Lipasekristall bei einem pH-Wert von 2.0 bis 9.0 aktiv ist.

2. Zusammensetzung nach Anspruch 1, wobei der Lipasekristall aktiv ist, nachdem er mindestens eine Stunde einer Umgebung ausgesetzt wurde, die einen pH-Wert von 1.0 bis 4.0 hat.

3. Zusammensetzung nach Anspruch 1, wobei der Lipasekristall aktiv ist, nachdem er mindestens zwei Stunden einer Umgebung ausgesetzt wurde, die einen pH-Wert von 1.0 bis 4.0 hat.

4. Zusammensetzung nach Anspruch 1, wobei der Lipasekristall aktiv ist, nachdem er mindestens fünf Stunden einer Umgebung ausgesetzt wurde, die einen pH-Wert von 1.0 bis 4.0 hat.

5. Zusammensetzung nach Anspruch 1, wobei das multifunktionale Vernetzungsmittel Bis-(sulfosuccinimidyl)-suberat ist.

6. Zusammensetzung nach Anspruch 1, wobei die Lipase von einer bakteriellen Lipase abgeleitet ist.

7. Zusammensetzung nach Anspruch 6, wobei die bakterielle Lipase eine *Pseudomonas-Lipase* ist.

8. Zusammensetzung nach Anspruch 7, wobei die *Pseudomonas-Lipase* eine *Burkholderia cepacia*-Lipase ist.

9. Zusammensetzung nach Anspruch 1, wobei die Amylase ausgewählt ist aus der Gruppe bestehend aus einer *Bacillus*-Amylase und einer *Aspergillus-*Amylase.

10. Zusammensetzung nach Anspruch 1, wobei die Protease eine von Pilzen stammende Protease ist.

11. Zusammensetzung nach Anspruch 1, wobie die Protease ein Proteasekristall ist.

12. Zusammensetzung nach Anspruch 11, wobei der Proteasekristall ein vernetzter Protease kristall ist.

13. Zusammensetzung nach Anspruch 1, wobei die Protease in einer nichtkristallinen Form ist.

14. Zusammensetzung nach Anspruch 1, wobei die Zusammensetzung in einer Pulverform ist.

15. Zusammensetzung nach Anspruch 1, wobei die Zusammensetzung eine wässrige Aufschlämmung ist.

16. Zusammensetzung nach Anspruch 1, wobei die Zusammensetzung in Form einer Kapsel oder Tablette ist.

17. Zusammensetzung nach Anspruch 1, wobei der Lipasekristall eine spezifische Aktivität für Olivenöl von mehr als 500, 1000, 4000, 5000, 6000, 7000, 8000, 9000 oder mehr Einheiten pro mg Lipase aufweist.

18. Zusammensetzung nach Anspruch 1, ferner umfassend einen pharmazeutisch verträglichen Träger.

19. Zusammensetzung nach Anspruch 1, wobei die Zusammensetzung ein Arzneimittel ist.

20. Zusammensetzung nach einem der Ansprüche 1, 18 oder 19, wobei die Zusammensetzung in einer Formulierung vorliegt, die geeignet ist, einem Individuum oral verabreicht zu werden.

21. Zusammensetzung nach Anspruch 18, wobei der pharmazeutische Träger ausgewählt ist aus der Gruppe bestehend aus einem Verdünnungsmittel, Exzipienten und Adjuvans.

22. Zusammensetzung nach Anspruch 18, wobei der Träger ein polymerer Träger ist.

23. Zusammensetzung nach Anspruch 22, wobei der polymere Träger ein biologisch abbaubares Polymer ist.

24. Zusammensetzung nach Anspruch 22, wobei die Zusammensetzung innerhalb einer Matrix des polymeren Trägers eingekapselt ist.

25. Zusammensetzung nach Anspruch 24, wobei mindestens 50% der Zusammensetzung innerhalb der Matrix eingekapselt bleibt, nachdem der polymere Träger für mindestens eine Stunde einer Umgebung ausgesetzt wurde, die einen pH-Wert von 1.0 bis 3.0 hat.

26. Verwendung der Zusammensetzung nach Anspruch 1 für die Herstellung eines Arzneimittels zur Behandlung oder Vorbeugung einer Magen-Darm-Störung in einem Säuger.

27. Verwendung nach Anspruch 26, wobei die Zusammensetzung oral zu verabreichen ist.

28. Verwendung nach Anspruch 26, wobei der Säuger ein Mensch ist.

29. Verwendung nach Anspruch 26, wobei die Magen-Darm-Störung ausgewählt ist aus der Gruppe bestehend aus Pankreatitis und Pankreasinsuffizienz.

30. Verwendung nach Anspruch 26, wobei das Individuum an cystischer Fibrose leidet oder gefährdet ist, an cystischer Fibrose zu erkranken.

31. Verwendung der Zusammensetzung nach Anspruch 1 für die Herstellung eines Arzneimittels zur Behandlung oder Vorbeugung von Fettresorptionsstörung in einem Säuger, der an einem Zustand leidet oder gefährdet ist, einen Zustand zu erleiden, der durch niedrige Lipasesekretion **gekennzeichnet** ist.

32. Verwendung nach Anspruch 31, wobei die Zusammensetzung dem Säuger oral zu verabreichen ist.

33. Verwendung nach Anspruch 31, wobei der Säuger ein Mensch ist.

34. Verwendung nach Anspruch 31, wobei die Zusammensetzung dem Individuum vor dem Essen zu verabreichen ist.

35. Verwendung nach Anspruch 31, wobei die Zusammensetzung dem Individuum zum Essen zu verabreichen ist.

36. Verwendung nach Anspruch 31, wobei die Zusammensetzung dem Individuum nach dem Essen zu verabreichen ist.

37. Verwendung nach Anspruch 31, wobei die Zusammensetzung dem Säuger in einer Menge zu verabreichen ist, die ausreicht, den Koeffizienten der Fettabsorption in dem Säuger um mehr als 60% zu erhöhen.

38. Verwendung nach Anspruch 31, wobei die Zusammensetzung dem Säuger in einer Menge zu verabreichen ist, die ausreicht, den Koeffizienten der Fettabsorption in dem Säuger um mehr als 80% zu erhöhen.

39. Verwendung nach Anspruch 26, wobei die Zusammensetzung dem Säuger in einer Menge zu verabreichen ist, die ausreicht, den Koeffizienten der Proteinabsorption in dem Säuger um mehr als 60% zu erhöhen.

## Revendications

1. Composition comprenant :
(a) un cristal de lipase non fongique réticulé avec un agent de réticulation multifonctionnel ;
(b) une protéase ; et
(c) une amylase, sous forme amorphe
dans laquelle le cristal de lipase est actif à un pH de 2,0 à 9,0.

2. Composition selon la revendication 1, dans laquelle le cristal de lipase est actif après exposition pendant au moins une heure à un environnement ayant un pH de 1,0 à 4,0.

3. Composition selon la revendication 1, dans laquelle le cristal de lipase est actif après exposition pendant au moins deux heures à un environnement ayant un pH de 1,0 à 4,0.

4. Composition selon la revendication 1, dans laquelle le cristal de lipase est actif après exposition pendant au moins cinq heures à un environnement ayant un pH de 1,0 à 4,0.

5. Composition selon la revendication 1 dans laquelle l'agent de réticulation multifonctionnel est le subérate de bis(sulfosuccinimidyle).

6. Composition selon la revendication 1 dans laquelle la lipase dérive d'une lipase bactérienne.

7. Composition selon la revendication 6, dans laquelle la lipase bactérienne est une lipase de *Pseudomonas.*

8. Composition selon la revendication 7, dans laquelle la lipase de *Pseudomonas* est la lipase de *Burkholderia cepacia.*

9. Composition selon la revendication 1, dans laquelle l'amylase est choisie dans le groupe constitué par une amylase de *Bacillus* et une amylase *d'Aspergillus.*

10. Composition selon la revendication 1, dans laquelle la protéase est une protéase fongique.

11. Composition selon la revendication 1, dans laquelle la protéase est un cristal de protéase.

12. Composition selon la revendication 11, dans laquelle le cristal de protéase est un cristal de protéase réticulé.

13. Composition selon la revendication 1 dans laquelle la protéase est sous forme non cristalline.

14. Composition selon la revendication 1, dans laquelle la composition est sous la forme d'une poudre.

15. Composition selon la revendication 1 dans laquelle la composition est une bouillie aqueuse.

16. Composition selon la revendication 1 ou dans laquelle la composition est sous la forme d'une capsule ou d'un comprimé.

17. Composition selon la revendication 1, dans laquelle le cristal de lipase présente une activité spécifique vis-à-vis de l'huile d'olive à plus de 500, 1 000, 4 000, 5 000, 6 000, 7 000, 8 000, 9 000 unités ou plus par mg de lipase.

18. Composition selon la revendication 1, comprenant en outre un véhicule acceptable sur le plan pharmaceutique.

19. Composition selon la revendication 1, laquelle composition est une composition pharmaceutique.

20. Composition selon l'une quelconque des revendications 1, 18 ou 19, laquelle composition est présente dans une formulation adaptée pour une délivrance par voie orale à un sujet.

21. Composition selon la revendication 18, dans laquelle le véhicule pharmaceutique est choisi dans le groupe constitué par un diluant, un excipient et un adjuvant.

22. Composition selon la revendication 18, dans laquelle le véhicule est un véhicule polymère.

23. Composition selon la revendication 22, dans laquelle le véhicule polymère est un polymère biodégradable.

24. Composition selon la revendication 22, laquelle composition est encapsulée à l'intérieur d'une matrice du véhicule polymère.

25. Composition selon la revendication 24, dans laquelle au moins 50 % de la composition restent encapsulés à l'intérieur de la matrice après exposition du véhicule polymère à un environnement ayant un pH de 1,0 à 3,0 pendant au moins une heure.

26. Utilisation de la composition selon la revendication 1 pour la préparation d'une composition pharmaceutique destinée à traiter ou à prévenir un trouble gastro-intestinal chez un mammifère.

27. Utilisation selon la revendication 26, dans laquelle la composition est destinée à être administrée par voie orale.

28. Utilisation selon la revendication 26, dans laquelle le mammifère est un humain.

29. Utilisation selon la revendication 26, dans laquelle le trouble gastro-intestinal est choisi dans le groupe constitué par la pancréatite et l'insuffisance pancréatique.

30. Utilisation selon la revendication 26, dans laquelle le sujet souffre ou risque de souffrir de mucoviscidose.

31. Utilisation de la composition selon la revendication 1 pour la préparation d'une composition pharmaceutique destinée à traiter ou à prévenir une mauvaise absorption des graisses chez un mammifère souffrant ou risquant de souffrir d'un état **caractérisé par** une faible sécrétion de lipase.

32. Utilisation selon la revendication 31, dans laquelle la composition est destinée à être administrée au mammifère par voie orale.

33. Utilisation selon la revendication 31, dans laquelle le mammifère est un humain.

34. Utilisation selon la revendication 31, dans laquelle la composition est destinée à être administrée au sujet de manière préprandiale.

35. Utilisation selon la revendication 31, dans laquelle la composition est destinée à être administrée au sujet de manière prandiale.

36. Utilisation selon la revendication 31, dans laquelle la composition est destinée à être administrée au sujet de manière postprandiale.

37. Utilisation selon la revendication 31, dans laquelle la composition est destinée à être administrée au mammifère en une quantité suffisante pour augmenter le coefficient d'absorption des graisses dans le mammifère à plus de 60 %.

38. Utilisation selon la revendication 31, dans laquelle la composition est destinée à être administrée au mammifère en une quantité suffisante pour augmenter le coefficient d'absorption des graisses dans le mammifère à plus de 80 %.

39. Utilisation selon la revendication 26, dans laquelle la composition est destinée à être administrée au mammifère en une quantité suffisante pour augmenter le coefficient d'absorption des protéines dans le mammifère à plus de 60 %.
